**Europäisches Patentamt**

**European Patent Office**

**Office européen des brevets**

(19)

(11) Veröffentlichungsnummer : **0 427 124 B1**

(12)

# EUROPÄISCHE PATENTSCHRIFT

(45) Veröffentlichungstag der Patentschrift :
31.03.93 Patentblatt 93/13

(51) Int. Cl.$^5$ : **A61K 9/20**, A61K 33/06, A61J 3/10

(21) Anmeldenummer : **90120999.9**

(22) Anmeldetag : **02.11.90**

(54) **Antacidatabletten.**

(30) Priorität : **10.11.89 DE 3937455**

(43) Veröffentlichungstag der Anmeldung :
**15.05.91 Patentblatt 91/20**

(45) Bekanntmachung des Hinweises auf die
Patenterteilung :
**31.03.93 Patentblatt 93/13**

(84) Benannte Vertragsstaaten :
**AT BE CH DE DK FR GB IT LI NL SE**

(56) Entgegenhaltungen :
**EP-A- 0 166 315**

(73) Patentinhaber : **NORDMARK ARZNEIMITTEL
GmbH.
Pinnauallee 4
W-2082 Uetersen (DE)**

(72) Erfinder : **Moest, Thomas, Dr.
An der Duene 9
W-2082 Moorrege (DE)**

(74) Vertreter : **Karau, Wolfgang, Dr. et al
BASF Aktiengesellschaft Carl-Bosch-Strasse
38
W-6700 Ludwigshafen (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft kleine Antacidatabletten, von denen pro Dosis 50 bis 1000 Stück mit Flüssigkeit geschluckt werden. Dadurch wird die Unannehmlichkeit der Einnahme üblicher Antacidaformen vermieden.

Arzneistoffe, die in hohen Dosierungen eingenommen werden müssen und unangenehme Einnahmeeigenschaften aufweisen, indem sie Ablagerungen auf und zwischen den Zähnen und im Mundraum verursachen, werden von den Patienten nur widerstrebend eingenommen (geringe Patientencompliance).

Beispiele hierfür sind Antacida, die häufig in Mengen von ca. 1 bis 3 g eingenommen werden und in Form von Kautabletten oder Gelen im Handel sind. Kautabletten verursachen beim Zerkauen nachhaltige Ablagerungen an den Zähnen, die wegen der sandigen Struktur der nichtlöslichen Wirkstoffe für längere Zeit in unangenehmer Weise erhalten bleiben. Gele erfordern eine umfangreiche Vorbereitung mit Aufreißen eines Beutels, Kneten und Herausdrücken des Geles, eventuell Einrühren in Wasser und/oder Applikation zum Mund. Auch hierbei setzen sich die unlöslichen Substanzen zwischen den Zähnen und im Mundraum ab.

Der Erfindung lag die Aufgabe zugrunde, eine Formgebung für Antacida zu entwickeln, die die Unannehmlichkeiten bei deren Einnahme vermeidet.

Die Lösung dieser Aufgabe besteht in bikonvexen Tabletten, deren Höhe und Durchmesser etwa gleich ist und 1,5 bis 4 mm beträgt, und die neben üblichen galenischen Hilfsstoffen ein Antacidum als Wirkstoff enthalten. Diese kleinen Tabletten sind leicht schluckbar und bleiben nicht im Mundraum hängen oder kleben.

Antacida sind Stoffe, die einer Hyperacidität des Magensaftes (Sodbrennen) entgegenwirken. Geeignete Verbindungen sind z.B. Magnesiumhydroxid, -oxid, -carbonat und -silikat, Aluminiumhydroxid, -phosphat, Magnesium-Aluminiumsilikate. Das früher verwendete Natriumbicarbonat wird nicht mehr eingesetzt. Die Antacida werden im allgemeinen in Mengen von 60 bis 95 Gew.-%, vorzugsweise 65 bis 90 Gew.-%, bezogen auf die Tablette (ohne Überzug), eingesetzt.

Die Tabletten werden mit üblichen galenischen Hilfsstoffen in Anteilen von 2 bis 20 % mit einem Durchmesser von 1,5 bis 4 mm in bikonvexer Form hergestellt, wobei die Höhe etwa gleich dem Durchmesser ist (±max. 25, vorzugsweise max. 10 %). Es brauchen nicht die ganzen Flächen konvex zu sein, vielmehr können sie - fertigungsbedingt - einen schmalen, flachen Rand haben.

Übliche galenische Hilfsstoffe sind z.B. Bindemittel wie Lactose, Stärke, Gelatine, mikrokristalline Cellulose oder Polyvinylpyrrolidon (PVP), Sprengmittel wie vernetztes PVP, Maisstärke, Stärkeglycolat oder Alginsäure, Gleitmittel wie Magnesiumstearat oder Talk, Fließmittel wie hochdisperses Siliziumdioxid.

Zur Vermeidung von Geschmacksirritationen und zur Verbesserung der Schluckbarkeit können die Tabletten in geläufigen Verfahren mit wasserlöslichen Polymeren oder Zucker überzogen werden (Filmtabletten, Dragees) Als Polymere werden bevorzugt wasserlösliche Cellulosederivate wie Methylcellulose oder Hydroxypropylmethylcellulose, ferner PVP eingesetzt.

50 bis 500 derartiger Tabletten entsprechender Größe und gewünschter Dosierung können als Granulat mit üblichen Dosiervorrichtungen wie Dosierlöffel oder Dosierabteilern oder auch fertig abgeteilt in Beutein zur Anwendung kommen. Nach Einnahme mit einer sinnvollen Menge Flüssigkeit verbleibt kein Rückstand im Mund.

Durch die Einbeziehung von Sprengmitteln in die Tablettenrezeptur kann sichergestellt werden, daß jede Tablette im Magen schnell zerfällt, so daß in Verbindung mit der großen Oberfläche und der Verteilung der Tabletten im Magenvolumen eine schnelle Wirkstoffverfügbarkeit gewährleistet ist.

Die kleinen Tabletten können beispielsweise gemäß EP-B 166 315 hergestellt werden.

EP 0 427 124 B1

Beispiel 1

Zusammensetzung

|  | | Dosiseinheit | Charge |
|---|---|---|---|
| 1. | Aluminiumhydroxid (getrocknetes Gel) | 600 mg | 1,20 kg |
| 2. | Magnesiumhydroxid (Pulver) | 210 mg | 0,42 kg |
| 3. | Lactose | 50 mg | 0,10 kg |
| 4. | Polyvinylpyrrolidon | 35 mg | 0,07 kg |
| 5. | Polyvinylpyrrolidon vernetzt | 40 mg | 0,08 kg |
| 6. | Hochdisperses Siliciumdioxid | 5 mg | 0,01 kg |
| 7. | Magnesiumstearat | 20 mg | 0,04 kg |

Herstellung von 2000 Dosiseinheiten

Die Bestandteile 1. bis 3. wurden in einem Labor-Hochleistungsmischer (Stephan UMC 12) gemischt und mit einer 8 % wäßrigen Lösung von 4. granuliert. Nach Passieren durch ein 1,0 mm Sieb wurde in einem Umlufttrockenschrank bei 60 bis 70°C getrocknet. Das trockene Granulat wurde durch ein 0,63 mm Sieb passiert und anschließend mit 5. und 6. zunächst vorgemischt und nach Zusatz von 7. fertiggemischt.

Die preßfertige Masse wurde auf einer Exzenterpresse mit 2,5 mm Stempeln mit konkaver Wölbung zu 2,5 mm hohen Mikrotabletten verpreßt mit einer Einzelmasse von 12,0 mg.

80 Stück dieser Mikrotabletten ergeben eine Dosiseinheit. Die Zerfallszeit (Methode Ph.Eur.) der Mikrotabletten beträgt 9 min, sie lassen sich bequem, ohne unangenehme Empfindungen mit einem Glas Wasser schlucken.

Beispiel 2

Zusammensetzung

|  | | Dosiseinheit | Charge |
|---|---|---|---|
| 1. | Aluminiumhydroxid (getrocknetes Gel) | 500 mg | 1,00 kg |
| 2. | Calciumcarbonat (schwer, Pulver) | 100 mg | 0,20 kg |
| 3. | Magnesiumhydroxid (Pulver) | 200 mg | 0,40 kg |
| 4. | Polyvinylpyrrolidon | 30 mg | 0,06 kg |
| 5. | Lactose, direkttablettierbar | 130 mg | 0,26 kg |
| 6. | Microkristalline Cellulose | 100 mg | 0,20 kg |
| 7. | Maisstärke | 100 mg | 0,20 kg |
| 8. | Hochdisperses Siliciumdioxid | 10 mg | 0,02 kg |
| 9. | Magnesiumstearat | 30 mg | 0,06 kg |
| 10. | Hydroxypropylmethylcellulose | 20 mg | 0,04 kg |

Herstellung von 2000 Dosiseinheiten

Die Bestandteile 1. bis 3. wurden in einem Labor-Hochleistungsmischer (Stephan UMC 12) gemischt und mit einer 8 % wäßrigen Lösung von 4. granuliert. Nach Passieren durch ein 1,2 mm Sieb wurde in einem Trockenschrank bei 60°C getrocknet. Das trockene Granulat wurde durch ein 0,8 mm Sieb passiert und anschließend mit 5. bis 8. zunächst vorgemischt und nach Zusatz von 9. fertiggemischt.

Die preßfertige Masse wurde auf einer Exzenterpresse mit 3 mm Stempeln mit konkaver Wölbung zu 3 mm hohen Mikrotabletten verpreßt mit einer Einzelmasse von 20 mg.

In einem Wirbelschichtgerät (Glatt WSG 5) wurde eine 5 % wäßrige Lösung von 10. bei 50°C Zulufttem-

3

peratur auf die Mikrotabletten aufgesprüht.

60 Stück dieser Mikrotabletten ergeben eine Dosiseinheit. Die Zerfallzeit (Methode Ph.Eur.) der Mikrotabletten beträgt 8 min. Sie lassen sich bequem ohne unangenehme Empfindungen mit einem Glas Wasser schlucken.

**Patentansprüche**

1.  Bikonvexe Tablette, deren Höhe und Durchmesser etwa gleich sind und 1,5 bis 4 mm betragen, und die neben üblichen galenischen Hilfsstoffen ein Antacidum als Wirkstoff enthält.

2.  Tablette nach Anspruch 1 mit 2 bis 3 mm Durchmesser.

**Claims**

1.  A biconvex tablet whose height and diameter are approximately equal and are from 1.5 to 4 mm and which, besides conventional pharmaceutical auxiliaries, contains an antacid as active substance.

2.  A tablet as claimed in claim 1 with a diameter of from 2 to 3 mm.

**Revendications**

1.  Tablette biconvexe, dont la hauteur et le diamètre sont à peu près égaux et sont de 1,5 à 4 mm et qui contient, outre des auxiliaires galéniques usuels, un antacide comme principe actif.

2.  Tablette selon la revendication 1 d'un diamètre de 2 à 3 mm.